# EUROPEAN PATENT SPECIFICATION

(11) **EP 2 114 398 B1**
(45) Date of publication and mention of the grant of the patent: **30.06.2010**
(21) Application number: 07858167.5
(22) Date of filing: 27.12.2007
(51) Int. Cl.: A61K 31/34, A61P 27/00, A61P 27/06, A61P 29/00, A61P 43/00

(54) **ISOSORBIDE MONONITRATE DERIVATIVES FOR THE TREATMENT OF OCULAR HYPERTENSION**
ISOSORBID-MONONITRAT-DERIVATE ZUR BEHANDLUNG VON OKULARER HYPERTONIE
DÉRIVÉS DE MONONITRATE D'ISOSORBIDE POUR LE TRAITEMENT DE L'HYPERTENSION OCULAIRE

(30) Priority: 28.12.2006 EP 06380338
(43) Date of publication of application: 11.11.2009
(73) Proprietor: LACER, S.A., 08025 Barcelona (ES)
(72) Inventor: REPOLLÉS MOLINER, José, 08025 Barcelona (ES); PUBILL COY, Francisco, 08025 Barcelona (ES); MOURELLE MANCINI, Marisabel, 08025 Barcelona (ES)
(74) Representative: ABG Patentes, S.L.
(86) International application number: PCT/EP2007/064570
(87) International publication number: WO 2008/080939

(56) References cited:
- EP-A1- 0 000 001
- EP-A1- 1 120 419
- WO-A-2005/037842
- NALLET J P ET AL: "SYNTHESIS OF A SERIES OF HEXITOL AND AMINODEOXY.HEXITOL MONO-NITRATE DERIVATIVES CONTAINING A SULFUR GROUP AND PHARMACOLOGICAL EVALUATION OF ISOLATED RAT AORTAS" EUROPEAN JOURNAL OF ORGANIC CHEMISTRY, WILEY-VCH VERLAG, WEINHEIM, DE, no. 5, 1998, pages 933-943, XP001204497 ISSN: 1434-193X

## Description

The present invention relates to disulfide, sulfide, sulfoxide and sulfone derivatives of dianhydrohexite mononitrate of formula (I), tautomers, pharmaceutically acceptable salts and solvates thereof for the prevention and/or treatment of ophthalmological diseases mediated by ocular hypertension.

### BACKGROUND

Glaucoma is a degenerative disease of the eye wherein the intraocular pressure is too high to permit normal eye function. Pressure is elevated because drainage of aqueous fluid from within the eye is impaired. As a result, damage may occur to the optic nerve head and result in irreversible loss of visual function. If untreated, glaucoma may eventually lead to blindness. The several morphologically or functionally distinct types of glaucoma are typically characterized by elevated intraocular pressure (IOP), i.e., the condition of elevated intraocular pressure without optic nerve head damage or characteristic glaucomatous visual field defects, which is considered to be causally related to the pathological course of the disease. Some patients with glaucomatous field loss have relatively low IOP. These normal tension or low tension glaucoma patients can also benefit from agents that lower or control IOP. If glaucoma or ocular hypertension is detected early and treated promptly with medications that effectively reduce elevated intraocular pressure, the loss of visual function or its progressive deterioration can generally be ameliorated.

Current treatments for glaucoma focus on reducing pressure in the eye by reducing the amount of aqueous fluid being produced or by enhancing the flow of fluid out of the eye by mechanical or other means. However, currently available drugs do not enhance or restore functioning of the natural drainage pathway.

Glaucoma patients may also suffer reduced blood flow to the optic nerve and neuronal tissue, diminished resistance of the nerve tissue to damage, and compliance of connective tissue surrounding and supporting the optic nerve. Current treatments of do not address any such factors. Only, one agent, Memantine, has been proved to be an agent that may increase the relative resistance of the nerve tissue to damage (i.e., neuroprotective).

The use of statins has been associated in some studies with a diminished risk of developing age-related macular degeneration. To the extent that excess total cholesterol or LDL cholesterol is implicated in this condition, use of statins would reduce the risk of developing, or at least delay the onset of, this condition. Many statins also inhibit the activity of rho-kinase, such inhibition has been shown to enhance aqueous outflow [Rao et al., Invest. Ophthalmol. & Vis. Sci. 42:1029-1037 (2001)]. There may be as yet undiscovered or indirect effects of these compounds that would help explain their protective associations.

Recently, potassium channel blockers were found to reduce intraocular pressure in the eye and therefore provide yet one more approach to the treatment of ocular hypertension and the degenerative ocular conditions related thereto. Blockage of potassium channels can diminish fluid secretion and, under some circumstances, increase smooth muscle contraction and would be expected to lower IOP and have neuroprotective effects in the eye [US5,573,758 and US5,925,342; Moore, et al., Invest. Ophthalmol. Vis. Sci., 38, 1997; WO89/10757, WO94/28900 and WO96/33719].

In addition, the combination of prostaglandin compounds of the F-series (PGF) with antimicrobial peptides has been used in the treatment of increased intraocular pressure, such as that caused by glaucoma and the reduction of ocular hypertension [US2006264353].

In spite of the different therapies that have been developed for lowering intraocular pressure, and especially glaucoma, many individuals do not respond well when treated with existing glaucoma therapies.

Disulfide, sulfide, sulfoxide and sulfone derivatives of dianhydrohexite mononitrate have been used or evaluated in various studies related to different pathological conditions mediated by defects in the NO pathway, such as cardiovascular disorders [WO00/20420 and WO2005/037842]. However, with all of these studies, there has been no recognition that these compounds are capable of being effective in the treatment of glaucoma and/or lowering intraocular pressure.

### BRIEF DESCRIPTION OF THE INVENTION

The inventors have found surprisingly that compounds of formula (I), and specially 2-acetylthioisosorbide-5-mononitrate, have potential therapeutic effect against ophthalmological diseases, more precisely glaucoma.

The new application of these compounds is based on the results obtained by *in vivo* experiments in animals subjected to different stimulus such as intra ocular pressure, wherein it has been observed that administration of compounds of formula (I) reduces significantly the adverse effect. Additionally, in the treatment of ocular hypertension, the results pointed out that the compounds of formula (I) can be used even at concentration 10-fold lower than those used with conventional medicaments. Therefore, these compounds have a great efficacy in the reduction of ocular hypertension caused by different stimulus.

Accordingly, the present invention relates to the use of a compound of formula (I) or a tautomer, a pharmaceutically acceptable salt or a solvate thereof: 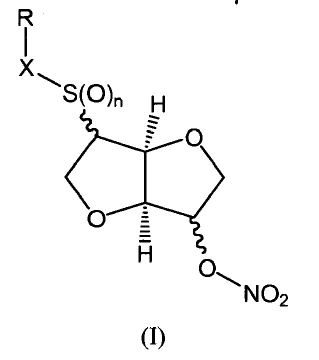 wherein:
n is an integer selected from 0, 1 and 2;
X is -S(O)ₘ-, -(C=O)- or a single bond, wherein m is an integer selected form 0, 1 and 2, with the proviso that when X is -(C=O)- then n is 0;
R is hydrogen or a residue R^{a}, wherein R^{a} is selected from the group consisting of:
   C₁₋₆ alkyl;
   C₂₋₆ alkenyl;
   C₃₋₈ cycloalkyl;
   C₃₋₈ cycloalkyl, wherein one CH₂ group is replaced by O, S, NH or NCH₃;
   C₄₋₈ cycloalkenyl;
   phenyl;
   pyridyl;
   thiophenyl;
   nitrosyl;
   S-cysteinyl;
   S-glutathionyl; and 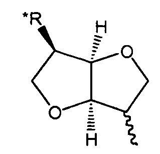 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
   and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,
as active ingredient(s) in the manufacture of a pharmaceutical composition for the prevention and/or treatment of ophthalmological diseases mediated by ocular hypertension.

In a particular embodiment, the ophthalmological disease is glaucoma, macular edema, age-related macular degeneration or diabetic retinopathy. In a preferred embodiment the ophthalmological disease is glaucoma.

In another aspect the invention relates to a compound of formula (I) as defined above for use in the treatment or prophylaxis of ophthalmological diseases mediated by ocular hypertension.

### DETAILED DESCRIPTION OF THE INVENTION

In the above definition of compounds of formula (I) used in the present invention, the following terms have the meaning indicated:
"C₁₋₆ alkyl" as used herein refers to a straight or branched hydrocarbon chain radical consisting of carbon and hydrogen atoms, containing no insaturation, having one to six carbon atoms, and which is attached to the rest of the molecule by a single bond, e. g., methyl, ethyl, *n*-propyl, *i*-propyl, *n*-butyl, *t*-butyl, *n*-pentyl.
"C₂₋₆ alkenyl" as used herein refers to a straight or branched chain alkenyl moiety consisting of carbon and hydrogen atoms, having one to six carbon atoms and at least one double bond of either E or Z stereochemistry where applicable, e.g., vinyl, allyl, 1- and 2-butenyl, and 2-methyl-2-propenyl.
"C₃₋₈ cycloalkyl" as used herein refers to an alicyclic group consisting of carbon and hydrogen atoms, having three to eight carbon atoms, e.g., cyclopropyl, cyclobutyl, cyclopentyl and cyclohexyl.

Accordingly, the term "C₃₋₈ cycloalkyl wherein one CH₂ group is replaced by O, S, NH or NCH₃" as used herein refers to an alicyclic group having from three to eight carbon atoms wherein one CH₂ group is replaced by O, S, NH o NCH₃, e.g., tetrahydropyrane, tetrahydrofurane, pyrrolidine, piperidine and tetrahydrothiophene.

"C₄₋₈ cycloalkenyl" as used herein refers to an alicyclic group consisting of carbon and hydrogen atoms, having four to eight carbon atoms, e.g., cyclopentenyl, cyclohexenyl, cycloheptenyl and cyclooctenyl.

"Halogen" as used herein refers to fluorine, chlorine, bromine or iodine, whereof bromine is preferred.

It is preferred that R represents hydrogen C₁₋₆ alkyl, C₂₋₆ alkenyl, C₃₋₈ cycloalkyl, C₄₋₈ cycloalkenyl, (C₁₋₆ alkyl)C₃₋₈ cycloalkyl, (C₁₋₆ alkyl)C₄₋₈ cycloalkenyl, phenyl or (C₁₋₆ alkyl)phenyl, whereas C₁₋₆ alkyl is especially preferred.

It is further preferred that in formula (I) either one or both ofm and n is 0.

Also it is preferred that X represents a single bond or -S-.

It is especially preferred that in the compounds of formula (I), RXS(O)ₙ- and ONO₂ are trans to each other with respect to the ring plane. The compound of formula (I) also include (R) and (S) diastereoisomers according to the formula (Ia) and (Ib):

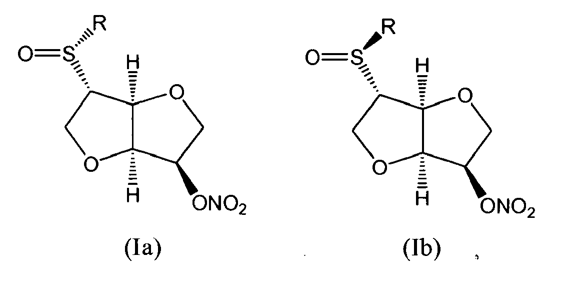

Especially preferred compounds of formula (I) are:
2-thioisosorbide 5-mononitrate;
5,5'-dinitrate-2,2'-dithiodiisosorbide;
2-methylthioisosorbide 5-mononitrate;
2-[(R)-methylsulfinyl]isosorbide 5-mononitrate;
2-[(S)-methylsulfinyl]isosorbide 5-mononitrate;
2-methylsulfinylisosorbide 5-mononitrate;
2-methylsulfonylisosorbide 5-mononitrate;
S-nitroso-2-thioisosorbide 5-mononitrate;
2-(tetrahydropyran-2-yl-thio) isosorbide 5-mononitrate;
2-(isosorbidyl-2'-dithio) isosorbide 5-mononitrate; and
2-(5'-acetyloxyisosorbidyl-2'-dithio) isosorbide 5-mononitrate.

Further it is especially preferred to use 2-acetylthio-isosorbide-5-mononitrate: 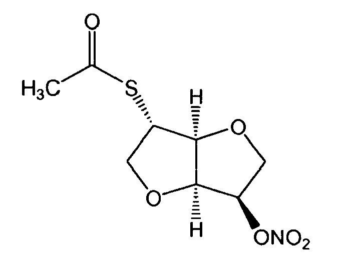 a tautomer, a pharmaceutically acceptable salt or a solvate thereof as active ingredient for the manufacture of a pharmaceutical composition for the prevention and/or treatment of opthalmological diseases mediated by ocular hypertension.

In a particular embodiment the ophthalmological disease is glaucoma.

Unless otherwise stated, the compounds used in the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, solvates" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts and derivatives can be carried out by methods known in the art.

For instance, pharmaceutically acceptable salts of compounds used in the invention are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favoured derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The compounds used in the present invention may be in crystalline form either as free compounds or as solvates (e.g. hydrates).

The compounds of formula (I) or their salts or solvates used in the invention are preferably in pharmaceutically acceptable or substantially pure form. By pharmaceutically acceptable form is meant, "inter alia", having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, or solvates.

The compounds used in the present invention represented by the formula (I) can include enantiomers, depending on the presence of chiral centers, and/or depending on the presence of multiple bonds (for example Z, E). The pure isomers, enantiomers or diastereoisomers and their mixtures are within the scope of the present invention.

The compounds of formula (I) used in the invention can be obtained by available synthetic procedures. Some examples of these procedures are described in WO2005/037842 and references therein.

In a particular embodiment of the present invention, the compounds of formula (I) for the treatment of ophthalmological diseases, are formulated in a suitable pharmaceutical composition, in a therapeutically effective quantity, together with one or more pharmaceutically acceptable carriers, adjuvants or excipients.

The pharmaceutical composition may be administered in the form of different preparations. Non limiting examples are preparations for oral administration, e.g. tablets, capsules, syrups or suspensions; ophthalmological administration, e.g. solutions, suspensions, ointments or creams; and parenteral administration, e.g. aqueous and non-aqueous sterile injection solutions or aqueous and non-aqueous sterile suspensions. Also, the pharmaceutical composition may include topical compositions, e.g. creams, ointments or pastes, or transdermic preparations such as patches or plasters. The pharmaceutical composition may also be prepared for vaginal or for rectal administration, e.g. rectal gel or suppository.

Generally an effective administered amount of a compound used in the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.01 to 100 mg/kg/day.

The compounds used in the present invention may also be administered with other drugs to provide a combination therapy. The other drugs may form part of the same composition, or be provided as a separate composition for administration at the same time or at different time.

In a particular embodiment the invention refers to the use of a compound of formula (I) or a tautomer, a pharmaceutically acceptable salt or a solvate thereof, for the manufacture of a pharmaceutical composition for the treatment and/or prevention of ophthamological diseases mediated by ocular hypertension. In a preferred embodiment, the ophthamological disease is glaucoma, macular edema, age-related macular degeneration or diabetic retinopathy, more preferably is glaucoma.

*In vivo* experiments in animals subjected to different stimulus such as high intraocular pressure have shown that administration of compounds of formula (I), reduce significantly the adverse effects. Additionally, in the case of the treatment of glaucoma, the results pointed out that the compounds of formula (I) can be used even at concentration 10-fold lower than those used with conventional medicaments. Therefore, these compounds have a great efficacy in the reduction of glaucoma caused by different stimulus.

### EXAMPLES

### Example 1. Results derived from the administration of 2-acetylthioisosorbide-5-mononitrate and 2-[(R)-methylsulfinyl] isosorbide-5-mononitrate in rabbits which have been subjected to alfa-chymotrypsin-induced glaucoma.

The method used was that described by Gabriele Campana, Claudio Bucolo, Giovanna Murari and Santi Spampinato in Pharmacol. Exp Therap Vol. 303, Issue 3, 1086-1094, December 2002

Animals were injected with intra muscular injection of dexamethasone at the dose rate of 10 mg/Kg body weight, to avoid immediate inflammation. Animals were anesthetized with ketamine (50 mg/kg IV) in combination with Dizepam.

Xylocaine (4%) was used for local anesthesia topically. A cannula attached to reservoir was inserted into the anterior chamber with the help of a 30 gauge needle to provide a hydrostatic pressure of 25 mmHg during injection of alpha-chymotrypsin. Then a second appropriately shaped 30 gauge needle was introduced near the pupil. Freshly prepared 150 units of alpha chymotrypsin prepared in 0.1 ml of sterile saline was irrigated through the cannula into the posterior chamber. Care was taken to prevent the contact of alpha chymotrypsin with corneal stroma. Both cannulas were carefully removed without significant loss of aqueous humor. Immediately after surgery the eye treated with Sofracort .(Corticosteroid) to reduce the chances of fungal or microbial infection.

All the animals were kept under observation for 5 days and after these five days the intraocular pressure (IOP) was measured daily with a Schiontz type indentation tonometer using 5.5 gm weights and by compilation of readings the maximum period required to achieve a stable increase in IOP was determined. It was found that 2 - 3 weeks were sufficient to achieve a stable increase in IOP. IOP was measured after 15 days for 3 consecutive days, every morning (at same time) to assure stable IOP. The rejection criteria in our study was the removal of those rabbits from the study which showed IOP< 30 mmHg.

### Treatment:

All the animals were closely observed for the development of glaucoma. Thirty animals showing the symptoms of glaucoma and with the intra ocular pressure more than 30 mmHg were selected for the present investigation.

Compounds 2-acetylthioisosorbide-5-mononitrate and 2-[(R)-methylsulfinyl] isosorbide-5-mononitrate (0.05%) were administered to two different groups of rabbits at a concentration of 0.05% by weight [0.05g in 100 mL of saline serum] by 3 drops instillation. For comparative data, the compound Timolol (Timoftol®) widely used for the treatment of glaucoma, was also administered to another group of rabbits at a concentration of 0.5% by weight.

The intraocular pressure measurements are included in the table 2 in mm Hg taken at 0, 1 and 2 h after 3 drops instillation.

| **Group** | **Treatment** | **0 hr** | **1 hr** | **2 hr** |
|---|---|---|---|---|
| Gr I | **trans-2-acetyl thioisosorbide-5-mononitrate (0.05%)** | 34.33 ± 0.68 | 24.72 ± 0.47 | 23.86 ± 0.32 |
| Gr II | **2-[(R)-methylsulfinyl] isosorbide-5-mononitrate (0.05%)** | 33.87 ± 0.38 | 23.51 ± 0.24 | 22.77 ± 0.19 |
| Gr III | **Timolol 0.5%** | 34.45 ± 0.58 | 24.77 ± 0.26 | 24.54 ± 0.26 |
| control | **Normal saline** | 33.76 ± 0.54 | 33.21 ± 0.34 | 32.37 ± 0.31 |

These results have pointed out that compounds 2-acetylthio-isosorbide-5-mononitrate and 2-[(R)-methylsulfinyl] isosorbide-5-mononitrate comprised in formula (I) significantly reduce the intraocular pressure showing similar results to those obtained with a conventional compound even when using 10-fold lower concentrations.

## Claims

1. A compound of formula (I) or a tautomer, a pharmaceutically acceptable salt, or a solvate thereof: 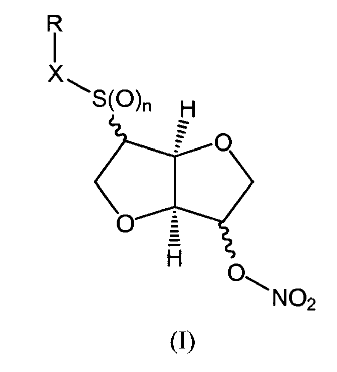 wherein:
n is an integer selected from 0, 1 and 2;
X is -S(O)ₘ-, -(C=O)- or a single bond, wherein m is an integer selected form 0, 1 and 2, with the proviso that when X is -(C=O)- then n is 0;
R is hydrogen or a residue R^{a}, wherein R^{a} is selected from the group :
C₁₋₆alkyl;
C₂₋₆ alkenyl;
C₃₋₈ cycloalkyl;
C₃₋₈ cycloalkyl, wherein one CH₂ group is replaced by O, S, NH or NCH₃;
C₄₋₈ cycloalkenyl;
phenyl;
pyridyl;
thiophenyl;
nitrosyl;
S-cysteinyl;
S-glutathionyl; and 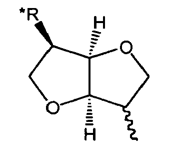
wherein R* is selected from hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,
for use in the prevention and/or treatment of ophthalmological diseases mediated by ocular hypertension.

2. A compound according to claim 1 wherein the ophthalmological disease is glaucoma, macular edema, age-related macular degeneration or diabetic retinopathy, more preferably is glaucoma.

3. A compound according to claims 1 or 2 wherein either one or both of m and n is 0.

4. A compound according to any one of claims 1 to 3 wherein X represents a single bond or -S-.

5. A compound according to any one of claims 1 to 4 wherein R is hydrogen C₁₋₆ alkyl, C₂₋₆ alkenyl, C₃₋₈ cycloalkyl, C₄₋₈ cycloalkenyl, (C₁₋₆ alkyl)C₃₋₈ cycloalkyl, (C₁₋₆ alkyl)C₄₋₈ cycloalkenyl, phenyl or (C₁₋₆ alkyl)phenyl.

6. A compound according to any one of claims 1 to 5 wherein R is C₁₋₆ alkyl.

7. A compound according to any one of claims 1 to 6 wherein the compound according to formula (I) is a compound of formula (Ia) or (Ib):

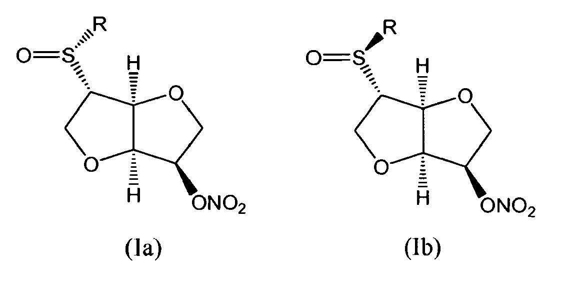

8. A compound according to any one of claims 1 to 7 wherein the compound of formula (I) is selected from:
2-thioisosorbide 5-mononitrate;
5,5'-dinitrate-2,2'-dithiodiisosorbide;
2-methylthioisosorbide 5-mononitrate;
2-[(R)-methylsulfinyl]isosorbide 5-mononitrate;
2-[(S)-methylsulfinyl]isosorbide 5-mononitrate;
2-methylsulfinylisosorbide 5-mononitrate;
2-methylsulfonylisosorbide 5-mononitrate;
S-nitroso-2-thioisosorbide 5-mononitrate;
2-(tetrahydropyran-2-yl-thio) isosorbide 5-mononitrate;
2-(isosorbidyl-2'-dithio) isosorbide 5-mononitrate; and
2-(5'-acetyloxyisosorbidyl-2'-dithio) isosorbide 5-mononitrate.

9. A compound according to claim 1 wherein the compound is 2-acetylthioisosorbide 5-mononitrate which is represented by the following formula:

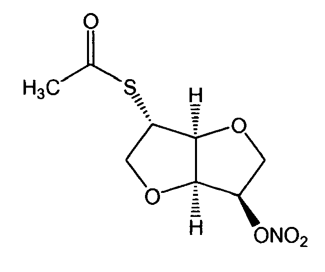

10. The use of a compound of formula (I) as defined in any of claims 1 or 3 to 9, as active ingredient in the manufacture of a pharmaceutical composition for the treatment and/or prophylaxis of ophthalmological diseases mediated by ocular hypertension.

11. The use according to claim 10 wherein the ophthalmological disease is glaucoma, macular edema, age-related macular degeneration or diabetic retinopathy, more preferably is glaucoma.

## Patentansprüche

1. Verbindung der Formel (I) oder ein Tautomer, ein pharmazeutisch akzeptables Salz oder ein Solvat davon: 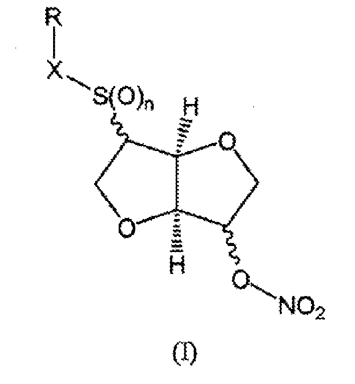 wobei:
n eine ganze Zahl aus 0, 1 und 2 ist;
X-S(O)ₘ-, -(C=O)- oder eine Einzelbindung ist, wobei m ein ganze Zahl aus 0, 1 und 2 ist, vorausgesetzt, dass, wenn X -(C=O)- ist, dann ist n = 0;
R Wasserstoff oder ein Rest R^{a} ist, wobei R^{a} aus der Gruppe ausgewählt ist:
C₁₋₆-Alkyl;
C₂₋₆-Alkenyl;
C₃₋₈-Cycloalkyl;
C₃₋₈-Cycloalkyl, wobei eine CH₂-Gruppe durch O, S, NH oder NCH₃ ersetzt ist;
C₄₋₈-Cycloalkenyl;
Phenyl;
Pyridyl;
Thiophenyl;
Nitrosyl;
S-Cysteinyl;
S-Glutathionyl; und 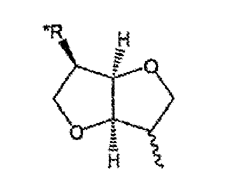
wobei R* ausgewählt ist aus Wasserstoff C, C₁₋₆-Alkyl, C₂₋₆-Alkenyl, C₃₋₈-Cycloalkyl, C₄₋₈-Cycloalkenyl, Acetyloxy, Hydroxyl, ONO₂ und Halogen; und wobei R^{a} wahlweise ersetzt wird durch ein bis drei Gruppen, die unabhängig ausgewählt sind aus C₁₋₆-Alkyl, C₂₋₆-Alkenyl, C₃₋₈-Cycloalkyl, C₄₋₈-Cycloalkenyl, Acetyloxy, Hydroxyl, ONO₂ und Halogen, für die Verwendung zur Prävention und/oder Behandlung ophthalmologischer Erkrankungen, die von okulärer Hypertension verursacht werden.

2. Verbindung nach Anspruch 1, wobei die ophthalmologische Erkrankung ein Glaukom, ein Makulaödem, altersbedingte Makuladegeneration oder diabetische Retinopathie, vorzugsweise aber ein Glaukom ist.

3. Verbindung nach Anspruch 1 oder 2, wobei entweder m oder n oder beides 0 ist.

4. Verbindung nach einem der Ansprüche 1 bis 3, wobei X eine Einfachbindung oder -S- repräsentiert.

5. Verbindung nach einem der Ansprüche 1 bis 4, wobei R Wasserstoff, ein C₁₋₆-Alkyl, C₂₋₆-Alkenyl, C₃₋₈-Cycloalkyl, C₄₋₈-Cycloalkenyl, (C₁₋₆-Alkyl)C₃₋₈ -Cykloalkyl, (C₁₋₆-Alkyl)C₄₋₈-Cycloalkenyl, Phenyl oder (C₁₋₆-Alkyl)Phenyl ist.

6. Verbindung nach einem der Ansprüche 1 bis 5, wobei R ein C₁₋₆-Alkyl ist.

7. Verbindung nach einem der Ansprüche 1 bis 6, wobei die Verbindung gemäß der Formel (I) eine Verbindung gemäß der Formel (Ia) oder (Ib) ist:

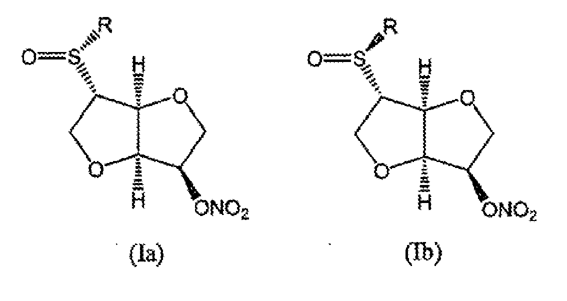

8. Verbindung nach einem der Ansprüche 1 bis 7, wobei die Verbindung der Formel (I) ausgewählt ist aus:
2-Thioisosorbid-5-Mononitrat;
5,5'-Dinitrat-2,2'-Dithiodiisosorbid;
2-Methylthioisosorbid-5-Mononitrat;
2-[(R)-Methylsulfinyl]Isosorbid-5-Mononitrat;
2-[(S)-Methylsulfinyl]Isosorbid-5-Mononitrat;
2-Methylsulfinylisosorbid-5-Mononitrat;
2-Methylsufonylisosorbid-5-Mononitrat;
S-Nitroso-2-Thioisosorbid-5-Mononitrat;
2-(Tetrahydropyran-2-yl-thio)Isosorbid-5-Mononitrat;
2-(Isosorbidyl-2'-Dithio)lsosorbid-5-Mononitrat; und
2-(5'-Acetyloxyisosorbidyl-2'-Dithio)Isosorbid-5-Mononitrat.

9. Verbindung nach Anspruch 1, wobei die Verbindung 2-Acetylthioisosorbid-5-Mononitrat ist, das durch die folgende Formel repräsentiert wird:

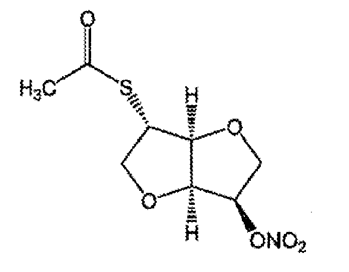

10. Verwendung einer Verbindung der Formel (I), wie in einem der Ansprüche 1 oder 3 bis 9 definiert, als Wirkstoff bei der Herstellung einer pharmazeutischen Zusammensetzung für die Behandlung und/oder Prophylaxe ophthalmologischer Erkrankungen, die durch okuläre Hypertension verursacht werden.

11. Verwendung nach Anspruch 10, wobei die ophthalmologische Erkrankung ein Glaukom, ein Makulaödem, altersbedingte Makuladegeneration oder diabetische Retinopathie, vorzugsweise aber ein Glaukom ist.

## Revendications

1. Composé de formule (I) ou tautomère, sel pharmaceutiquement acceptable ou solvate de celui-ci : 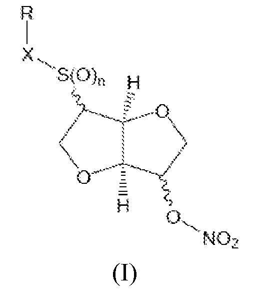 où :
n est un nombre entier sélectionné parmi 0, 1 et 2 ;
X est -S(O)ₘ-, -(C=O)- ou une liaison simple, où m est un nombre entier sélectionné parmi 0, 1 et 2, à condition que lorsque X est -(C=O)- n soit alors égal à 0 ;
R est un atome d'hydrogène ou un résidu R^{a}, dans lequel R^{a} est sélectionné dans le groupe :
alkyle en C₁₋₆ ;
alcényle en C₂₋₆ ;
cycloalkyle en C₃₋₈ ;
cycloalkyle en C₃₋₈, dans lequel un groupe CH₂ est remplacé par O, S, NH ou NCH₃ ;
cycloalcényle en C₄₋₈ ;
phényle ;
pyridyle ;
thiophényle ;
nitrosyle ;
S-cystéinyle ;
S-glutathionyle ; et 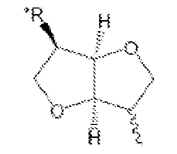 où R* est sélectionné parmi un atome d'hydrogène, un groupe alkyle en C₁₋₆, alcényle en C₂₋₆, cycloalkyle en C₃₋₈, cycloalcényle en C₄₋₈, acétyloxy, hydroxyle, ONO₂ et un atome d'halogène ;
et où R^{a} est facultativement substitué par un à trois groupes sélectionnés indépendamment parmi un groupe alkyle en C₁₋₆, alcényle en C₂₋₆, cycloalkyle en C₃₋₈, cycloalcényle en C₄₋₈, acétyloxy, hydroxyle, ONO₂ et un atome d'halogène,
pour l'utilisation dans la prévention et/ou le traitement de maladies ophtalmologiques médiées par l'hypertension oculaire.

2. Composé selon la revendication 1, dans lequel la maladie ophtalmologique est le glaucome, l'oedème maculaire, la dégénérescence maculaire liée à l'âge ou la rétinopathie diabétique, plus préférablement le glaucome.

3. Composé selon les revendications 1 ou 2, dans lequel soit m, soit n, soit les deux, est égal à 0.

4. Composé selon l'une quelconque des revendications 1 à 3, dans lequel X représente une liaison simple ou -S-.

5. Composé selon l'une quelconque des revendications 1 à 4, dans lequel R est hydrogène, alkyle en C₁₋₆, alcényle en C₂₋₆, cycloalkyle en C₃₋₈, cycloalcényle en C₄₋₈, (alkyle en C₁₋₆)-cycloalkyle en C₃₋₈, (alkyle en C₁₋₆)-cycloalcényle en C₄₋₈, phényle ou (alkyle en C₁₋₆)phényle.

6. Composé selon l'une quelconque des revendications 1 à 5, dans lequel R est un groupe alkyle en C₁₋₆.

7. Composé selon l'une quelconque des revendications 1 à 6, dans lequel le composé selon la formule (I) est un composé de formule (Ia) ou (Ib) :

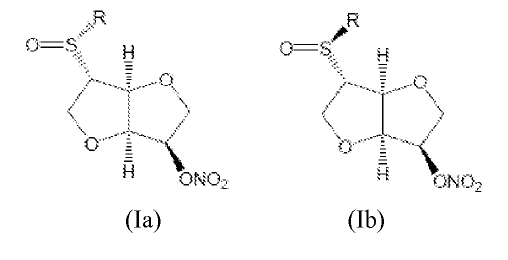

8. Composé selon l'une quelconque des revendications 1 à 7, dans lequel le composé de formule (I) est sélectionné parmi :
le 5-mononitrate de 2-thioisosorbide ;
le 5,5'-dinitrate-2, 2'-dithiodiisosorbide ;
le 5-mononitrate de 2-méthylthioisosorbide ;
le 5-mononitrate de 2-[(R)-méthylsulfinyl]isosorbide ;
le 5-mononitrate de 2-[(S)-méthylsulfmyl]isosorbide ;
le 5-mononitrate de 2-méthylsulfinylisosorbide ;
le 5-mononitrate de 2-méthylsulfonylisosorbide ;
le 5-mononitrate de S-nitroso-2-thioisosorbide ;
le 5-mononitrate de 2-(tétrahydropyran-2-yl-thio)isosorbide ;
le 5-mononitrate de 2-(isosorbidyl-2'-dithio)isosorbide ; et
le 5-mononitrate de 2-(5'-acétyloxyisosorbidyl-2'-dithio)isosorbide.

9. Composé selon la revendication 1, dans lequel le composé est le 5-mononitrate de 2-acétylthioisosorbide qui est représenté par la formule suivante :

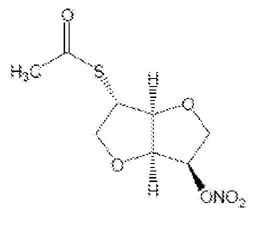

10. Utilisation d'un composé de formule (I) selon l'une quelconque des revendications 1 ou 3 à 9, en tant que principe actif dans la fabrication d'une composition pharmaceutique destinée au traitement et/ou à la prophylaxie de maladies ophtalmologiques médiées par l'hypertension oculaire.

11. Utilisation selon la revendication 10, dans laquelle la maladie ophtalmologique est le glaucome, l'oedème maculaire, la dégénérescence maculaire liée à l'âge ou la rétinopathie diabétique, plus préférablement le glaucome.
